# EUROPÄISCHE PATENTANMELDUNG

(11) **EP 2 330 140 A1**
(43) Veröffentlichungstag der Anmeldung: **08.06.2011**
(21) Anmeldenummer: 09177687.2
(22) Anmeldetag: 02.12.2009
(51) Int. Cl.: C08G 18/32, C07C 251/08, C09D 9/00, C11D 1/40, C11D 3/37, C11D 7/32, C11D 11/00

(54) **Aldimin - Reinigungszusammensetzung für reaktive Polyurethanzusammensetzungen**

(71) Anmelder: Sika Technology AG, 6340 Baar (CH)
(72) Erfinder: Janke, Doreen, 25486 Alveslohe (DE); Paschkowski, Kai, 21635 Jork (DE)
(74) Vertreter: Isler, Jörg

(57) **Zusammenfassung**

Die vorliegende Erfindung betrifft die Verwendung eines Aldimins der Formel (I) als Reinigungsmittel oder als Bestandteil einer Reinigungsmittelzusammensetzung für reaktive Polyurethanzusammensetzungen, insbesondere für reaktive Polyurethanheissschmelzklebstoffe.

Es konnte festgestellt werden, dass mit derartigen Reinigungsmitteln die Menge von Abfall stark reduziert werden konnte und dass insbesondere Mischungen von blockierten und nicht blockierten reaktive Polyurethanzusammensetzungen problemlos für Verklebungen verwendet werden konnte, ohne dass die Verklebungen negativ in ihren Eigenschaften, insbesondere der Wärmebeständigkeit, beeinflusst würden.

## Beschreibung

### Technisches Gebiet

Die vorliegende Erfindung betrifft Reinigungsmittel für die Reinigung von Herstell- und Verarbeitungsanlagen von reaktiven Polyurethanzusammensetzungen, insbesondere von reaktiven Polyurethan-Heissschmelzklebstoffen.

### Stand der Technik

Bei der Verarbeitung von reaktiven Polyurethanzusammensetzungen im Allgemeinen, und von reaktiven Polyurethan-Heissschmelzklebstoffen im Speziellen, treten aufgrund der Reaktivität mit Wasser, insbesondere in Form von Luftfeuchtigkeit, mit der Zeit stets ausgehärtete Klebstoffpartikel auf, welche sich nachteilig auf die Qualität und Applikation auswirken. Deshalb ist eine regelmässige Reinigung der Anlagen und Geräte nötig. Eine manuelle Reinigung ist jedoch sehr aufwendig, arbeitsintensiv und sehr kostspielig, da sie meist ein Auseinanderbauen der Apparatur bedingt. Eine weitere Schwierigkeit in der Verarbeitung besteht vor allem bei Betriebsunterbrüchen, wie beispielsweise bei kurzfristigen Pannen oder bei längeren Stillständen über Nacht oder über das Wochenende. In diesen Fällen verbleibt die PolyurethanZusammensetzung unbewegt in der Anlage und es können sich Aushärtungen an Oberflächen, insbesondere an beweglichen Teilen oder Düsen der Maschinen absetzen. Dies ist besonders dort kritisch, wo der Klebstoff mit Umgebungsluft in Kontakt tritt.

Aus diesem Grund ist es ein Bedürfnis, eine Alternative zur aufwendigen manuellen Reinigung zur Verfügung zu stellen. Es wurden Reinigungspasten vorgeschlagen, die aber aufgrund der darin enthaltenen abrasiven Bestandteilen die Anlagen stark schädigen und deshalb nicht eine wirkliche Alternative darstellen. EP 0 544 058 A2 offenbart eine Reinigungsmittelzusammensetzung, welche neben einer nicht Isocyanat-reaktiven Polyurethan-Masse einen monofunktionellen Alkohol beinhaltet.

Weiterhin offenbart US 5,772,790 eine Reinigungsmittelzusammensetzung, welchen neben einem nicht-härtbaren Polyurethanprepolymer ein monofunktionelles Amin beinhaltet. Amine sind jedoch arbeitshygienisch und umwelttoxikologisch problematisch, da Amine korrosiv wirken. Insbesondere die bevorzugten Fettamine sind problematisch, da sie im Falle eines unbeabsichtigten Kontaktes, beispielsweise bei einem Unfall, mit den Augen aufgrund ihres Tensidcharakters äusserst schwierig daraus zu entfernen sind, insbesondere bevor die ätzende Wirkung eintrifft. Weiterhin ist das Arbeiten mit niedermolekularen Aminen stets mit einer unangenehmen Geruchsentwicklung verbunden.

WO 2003-0018 offenbart eine Reinigungszusammensetzung basierend auf einem Amid oder einem monofunktionellen Imid.

All diese Ansatzpunkte des Standes der Technik basieren auf einer irreversiblen Blockierung der reaktiven Polyurethanzusammensetzung und weisen den grossen Nachteil, dass nach der Reinigung die über das Reinigungsmittel blockierte Polyurethanzusammensetzung vollständig entfernt werden muss, um Klebeprobleme, insbesondere in Bezug auf Wärmestabilität der Verklebung, zu verhindern. Um derartige Probleme zu verhindern, muss einerseits ein Prozesskontrollsystem gewährleistet werden und andererseits die blockierte Polyurethanzusammensetzung vor der Wiederaufnahme durch neuen reaktiven Polyurethanzusammensetzung praktisch vollständig ausgestossen werden, so dass nicht nur die so blockierte Polyurethanzusammensetzung sondern auch die Mischfraktionen mit neuer reaktiven Polyurethanzusammensetzung entsorgt werden muss. Die so zu entsorgende Menge von Klebstoff kann je nach Reiniger bzw. Anlage beträchtlich sein, was ökonomisch und ökologisch natürlich sehr nachteilig ist.

### Darstellung der Erfindung

Überraschenderweise hat sich gezeigt, dass diese Aufgabe durch die Verwendung eines Aldimins gemäss Anspruch 1 gelöst werden kann.

Aufgabe der vorliegenden Erfindung ist es daher, eine Reinigungsmittelzusammensetzung für reaktive Polyurethanzusammensetzungen zur Verfügung zustellen, welche eine Reinigung ermöglicht, bei welcher die über Reinigungsmittelzusammensetzung blockierte Polyurethanzusammensetzung nicht vollständig entsorgt werden muss, sondern dass auch Mischfraktionen für verlässliche wärmestabile Verklebungen verwendbar sind.

Insbesondere wird zwar durch diese spezifische Reinigungsmittelzusammensetzung die reaktive Polyurethanzusammensetzung blockiert, andererseits wird nach der Reinigung die so blockierte Reinigungsmittelzusammensetzung in einer Mischfraktion bei der Aushärtung mittels Feuchtigkeit der reaktiven Polyurethanzusammensetzung unter Aldehyd-Abspaltung wieder aktiv und wird ins Netzwerk der mit Feuchtigkeit ausgehärteten Polyurethanzusammensetzung eingebaut. Eine derartig ausgehärtete Polyurethanzusammensetzung weist im Wesentlichen die gleichen mechanischen Eigenschaften auf, wie wenn sie vor der Aushärtung nicht in Kontakt mit der Reinigungsmittelzusammensetzung getreten worden wäre.

Insbesondere zeigte sich, dass allfällig der Anlage nach der Reinigung entnommenen Fraktionen problemlos mit der reaktive Polyurethanzusammensetzung gemischt werden konnten und der Herstell- und Verarbeitungsanlagen wieder zugeführt werden konnten, ohne dass die Eigenschaften, insbesondere die Wärmestabilität, der damit hergestellten Verklebungen hierunter negativ beeinflusst würden. Somit können Reinigungen durchgeführt werden, die faktisch ohne ein Entsorgen von Materialien auskommen. Das Fehlen bzw. die zumindest stark reduzierten Mengen von Abfällen sind ökonomisch und ökologisch besonders interessant.

Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Die vorliegende Erfindung betrifft in einem ersten Aspekt die Verwendung eines Aldimins der Formel (I) als Reinigungsmittel oder als Bestandteil einer Reinigungsmittelzusammensetzung für reaktive Polyurethanzusammensetzungen, insbesondere für reaktive Polyurethanheissschmelzklebstoffe.

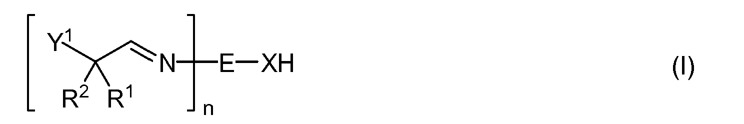

Hierbei stehen
R¹ und R² entweder unabhängig von einander jeweils für einen einwertigen Kohlenwasserstoffrest mit 1 bis 12 C-Atomen,
oder
zusammen für einen zweiwertigen Kohlenwasserstoffrest mit 4 bis 12 C-Atomen, der Teil eines, gegebenenfalls substituierten, carbocyclischen Rings mit 5 bis 8, bevorzugt 6, C-Atomen ist;
Weiterhin steht Y¹ für einen einwertigen Kohlenwasserstoffrest mit 1 bis 32 C-Atomen, welcher gegebenenfalls mindestens ein Heteroatom,
insbesondere Sauerstoff in der Form von Ether-, Carbonyl- oder Ester-Gruppen aufweist; und
E steht für einen (n+1)-wertigen Kohlenwasserstoffrest mit 2 bis 12 C-Atomen, welcher gegebenenfalls mindestens ein Heteroatom, insbesondere in Form von Ether-Sauerstoff oder tertiärem Amin-Stickstoff, aufweist;
X steht für O, S oder N-R⁸
wobei
R⁸ entweder
für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureestergruppe aufweist, steht,
oder
für einen Substituenten der Formel (II) steht 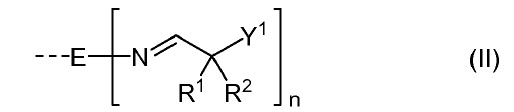 und n steht für 1, 2 oder 3.

Unter dem Term "reaktive Polyurethanzusammensetzung" wird im vorliegenden Dokument eine Polyurethanzusammensetzung bezeichnet, welche freie Isocyanatgruppen, insbesondere in Form von Isocyanatgruppen aufweisende Polyurethanpolymeren, aufweisen. Isocyanatgruppen sind "reaktiv", d.h. sie reagieren mit Wasser.

Der Begriff "Polyurethanpolymer" umfasst sämtliche Polymere, welche nach dem sogenannten Diisocyanat-Polyadditions-Verfahren hergestellt werden. Solche Polyurethanpolymere können neben Urethan- oder Thiourethangruppen insbesondere auch Harnstoffgruppen aufweisen.

Der Begriff "Polymer" umfasst im vorliegenden Dokument einerseits ein Kollektiv von chemisch einheitlichen, sich aber in Bezug auf Polymerisationsgrad, Molmasse und Kettenlänge unterscheidenden Makromolekülen, das durch eine Polyreaktion (Polymerisation, Polyaddition, Polykondensation) hergestellt wurde. Der Begriff umfasst andererseits auch Derivate eines solchen Kollektivs von Makromolekülen aus Polyreaktionen, Verbindungen also, die durch Umsetzungen, wie beispielsweise Additionen oder Substitutionen, von funktionellen Gruppen an vorgegebenen Makromolekülen erhalten wurden und die chemisch einheitlich oder chemisch uneinheitlich sein können. Der Begriff umfasst im Weiteren auch sogenannte Prepolymere, das heisst reaktive oligomere Voraddukte, deren funktionelle Gruppen am Aufbau von Makromolekülen beteiligt sind.

Mit "Poly" beginnende Substanznamen wie Polyaldimin, Polyamin, Polyol oder Polyisocyanat bezeichnen im vorliegenden Dokument Substanzen, die formal zwei oder mehr der in ihrem Namen vorkommenden funktionellen Gruppen pro Molekül enthalten.

Die gestrichelten Linien in den Formeln in diesem Dokument stellen jeweils die Bindung zwischen einem Substituenten und dem zugehörigen Molekülrest dar.

Der Begriff "primäre Aminogruppe" bezeichnet im vorliegenden Dokument eine Aminogruppe in der Form einer NH₂-Gruppe, die an einen organischen Rest gebunden ist. Der Begriff "sekundäre Aminogruppe" bezeichnet eine Aminogruppe, in der das Stickstoffatom an zwei organische Reste gebunden ist, welche auch gemeinsam Teil eines Rings sein können. Der Begriff "tertiäre Aminogruppe" bezeichnet eine Aminogruppe, in der das Stickstoffatom (= tertiärer Amin-Stickstoff) an drei organische Reste gebunden ist, wobei zwei dieser Reste auch gemeinsam Teil eines Rings sein können.

Der Begriff "aktiver Wasserstoff" bezeichnet im vorliegenden Dokument das Wasserstoffatom einer Hydroxyl-, einer Mercapto- oder einer sekundären oder primären Aminogruppe.

Als "aliphatisch" wird ein Amin und ein Isocyanat bezeichnet, deren Amino- und Isocyanatgruppen jeweils ausschliesslich an aliphatische, cycloaliphatische oder arylaliphatische Reste gebunden sind; entsprechend werden diese Gruppen als aliphatische Amino- und Isocyanatgruppen bezeichnet.

Als "aromatisch" werden ein Amin und ein Isocyanat bezeichnet, deren Amino- und Isocyanatgruppen jeweils an einen aromatischen Rest gebunden sind; entsprechend werden diese Gruppen als aromatische Amino- und Isocyanatgruppen bezeichnet.

Unter einer "geruchsarmen" Substanz wird eine Substanz verstanden, deren Geruch von menschlichen Individuen nur in geringem Masse wahrnehmbar, d.h. riechbar, ist, die also keinen intensiven Geruch aufweist wie beispielsweise Formaldehyd, Acetaldehyd, Isobutyraldehyd, oder Lösemittel wie Aceton, Methylethylketon oder Methylisobutylketon, und wobei dieser geringe Geruch von den meisten menschlichen Individuen nicht als unangenehm oder abstossend empfunden wird.

Unter einer "geruchsfreien" Substanz wird eine Substanz verstanden, die für die meisten menschlichen Individuen nicht riechbar ist, die also keinen wahrnehmbaren Geruch aufweist.

Als "Raumtemperatur" wird im vorliegenden Dokument eine Temperatur im Bereich von 20°C bis 25 °C bezeichnet.

Bevorzugt stehen R¹ und R² je für einen Methylrest.

Bevorzugt steht n für den Wert 1.

Weiterhin bevorzugt steht Y¹ für einen Rest der Formel (III a) oder (III b). 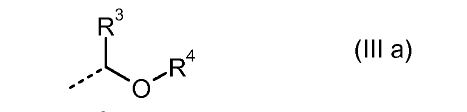 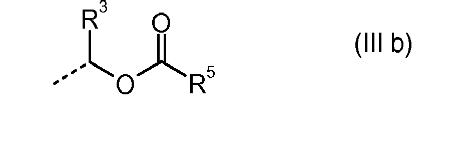 Hierbei steht R³ für ein Wasserstoffatom oder für einen Alkyl-, Cycloalkyl-, Arylalkyl- oder Alkoxycarbonylrest mit 1 bis 12 C-Atomen;
R⁴ steht für einen Kohlenwasserstoffrest mit 1 bis 30 C-Atomen, welcher gegebenenfalls Ethersauerstoffatome enthält.
Weiterhin steht R⁵ entweder
für ein Wasserstoffatom,
oder
für einen linearen oder verzweigten Alkylrest mit 1 bis 30 C-Atomen, gegebenenfalls mit cyclischen Anteilen und gegebenenfalls mit mindestens einem Heteroatom, insbesondere Sauerstoff in der Form von Ether-, Carbonyl- oder Ester-Gruppen,
oder
für einen einwertigen einfach oder mehrfach ungesättigten, linearen oder verzweigten Kohlenwasserstoffrest mit 5 bis 30 C-Atomen,
oder
für einen, gegebenenfalls substituierten, aromatischen oder heteroaromatischen 5- oder 6-gliedrigen Ring.

Das Aldimin der Formel (I) ist herstellbar aus mindestens einem sterisch gehinderten aliphatischen Aldehyd A und mindestens einem aliphatischen Amin **B**, entsprechend der Formel [H₂N]ₙ-E-XH, welches neben einer oder mehreren primären Aminogruppen noch eine weitere Reaktivgruppe , welche eine Hydroxylgruppe, eine Mercaptogruppe oder NH-R⁸ ist.

Die Umsetzung zwischen dem Aldehyd **A** und dem Amin **B** erfolgt in einer Kondensationsreaktion unter Abspaltung von Wasser. Solche Kondensationsreaktionen sind bestens bekannt und beschrieben, beispielsweise in Houben-Weyl, "Methoden der organischen Chemie", Vol. XI/2, Seite 73ff. Der Aldehyd **A** wird hierbei in Bezug auf die primären Aminogruppen des Amins **B** stöchiometrisch oder in stöchiometrischem Überschuss eingesetzt. Üblicherweise werden solche Kondensationsreaktionen in Anwesenheit eines Lösemittels durchgeführt, mittels welchem das bei der Reaktion entstehende Wasser azeotrop entfernt wird. Zur Herstellung der Aldimine der Formel (I) wird jedoch ein Herstellverfahren ohne Verwendung von Lösemitteln bevorzugt, wobei das bei der Kondensation gebildete Wasser direkt mittels Anlegen von Vakuum aus der Reaktionsmischung entfernt wird. Durch die lösemittelfreie Herstellung erübrigt sich ein Abdestillieren des Lösemittels nach erfolgter Herstellung, was den Herstellungsprozess vereinfacht. Zudem ist das Aldimin so frei von Lösemittelrückständen, welche einen störenden Geruch verursachen könnten.

Für die Herstellung des Aldimins der Formel (I) wird mindestens ein sterisch gehinderter aliphatischer Aldehyd A der Formel (IV) eingesetzt.

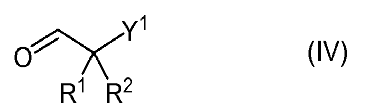

Der Aldehyd **A** ist vorzugsweise geruchsfrei. Unter einer "geruchsfreien" Substanz wird eine Substanz verstanden, die so geruchsarm ist, dass sie für die meisten menschlichen Individuen nicht riechbar, das heisst mit der Nase nicht wahrnehmbar, ist.

Aldehyde **A** der Formel (IV) sind tertiäre aliphatische oder tertiäre cycloaliphatische Aldehyde, wie beispielsweise Pivalaldehyd (= 2,2-Dimethylpropanal), 2,2-Dimethyl-butanal, 2,2-Diethyl-butanal, 1-Methyl-cyclopentancarboxaldehyd, 1-Methyl-cyclohexancarboxaldehyd; sowie Ether aus 2-Hydroxy-2-methylpropanal und Alkoholen wie Propanol, Isopropanol, Butanol und 2-Ethylhexanol; Ester aus 2-Formyl-2-methylpropionsäure oder 3-Formyl-3-methylbuttersäure und Alkoholen wie Propanol, Isopropanol, Butanol und 2-Ethylhexanol; Ester aus 2-Hydroxy-2-methylpropanal und Carbonsäuren wie Buttersäure, Isobuttersäure und 2-Ethylhexansäure; sowie die im folgenden als besonders geeignet beschriebenen Ether und Ester von 2,2-disubstituierten 3-Hydroxypropanalen, -butanalen oder analogen höheren Aldehyden, insbesondere von 2,2-Dimethyl-3-hydroxypropanal.

Besonders geeignete Aldehyde der Formel (IV) sind in einer ersten Ausführungsform Aldehyde der Formel (IV a).

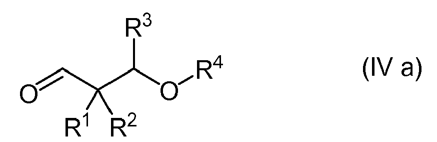

Aldehyde der Formel (IV a) stellen Ether von aliphatischen, araliphatischen oder cycloaliphatischen 2,2-disubstituierten 3-Hydroxyaldehyden mit Alkoholen der Formel HO-R⁴ dar, beispielsweise Fettalkoholen. Geeignete 2,2-disubstituierte 3-Hydroxyaldehyde sind ihrerseits erhältlich aus Aldol-Reaktionen, insbesondere gekreuzten Aldol-Reaktionen, zwischen primären oder sekundären aliphatischen Aldehyden, insbesondere Formaldehyd, und sekundären aliphatischen, sekundären araliphatischen oder sekundären cycloaliphatischen Aldehyden, wie beispielsweise 2-Methylbutyraldehyd, 2-Ethylbutyraldehyd, 2-Methylvaleraldehyd, 2-Ethylcapronaldehyd, Cyclopentancarboxaldehyd, Cyclohexancarboxaldehyd, 1,2,3,6-Tetrahydrobenzaldehyd, 2-Methyl-3-phenylpropionaldehyd, 2-Phenylpropionaldehyd (Hydratropaldehyd) oder Diphenylacetaldehyd.

Als besonders bevorzugte Aldehyde der Formel (IV a) gelten 2,2-Dimethyl-3-(2-ethylhexyloxy)-propanal, 2,2-Dimethyl-3-lauroxy-propanal und 2,2-Dimethyl-3-stearoxy-propanal.

In einer zweiten Ausführungsform sind besonders geeignete Aldehyde der Formel (IV) Aldehyde der Formel (IV b).

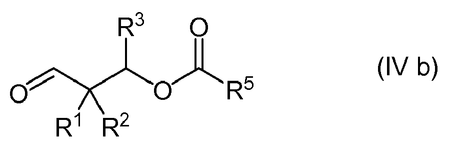

Verbindungen der Formel (IV b) stellen Ester der bereits beschriebenen 2,2-disubstituierten 3-Hydroxyaldehyde, wie beispielsweise 2,2-Dimethyl-3-hydroxypropanal, 2-Hydroxymethyl-2-methyl-butanal, 2-Hydroxymethyl-2-ethyl-butanal, 2-Hydroxymethyl-2-methyl-pentanal, 2-Hydroxymethyl-2-ethyl-hexanal, 1-Hydroxymethyl-cyclopentancarboxaldehyd, 1-Hydroxymethyl-cyclohexancarboxaldehyd 1-Hydroxymethyl-cyclohex-3-encarboxaldehyd, 2-Hydroxymethyl-2-methyl-3-phenyl-propanal, 3-Hydroxy-2-methyl-2-phenyl-propanal und 3-Hydroxy-2,2-diphenyl-propanal, mit geeigneten Carbonsäuren R⁵COOH dar.

Beispiele für geeignete Carbonsäuren R⁵COOH sind zum einen aliphatische Carbonsäuren, wie Ameisensäure, Essigsäure, Propionsäure, Buttersäure, Isobuttersäure, Valeriansäure, Capronsäure, 2-Ethyl-capronsäure, Caprinsäure, Laurinsäure, Tridecansäure, Myristinsäure, Pentadecansäure, Palmitinsäure, Margarinsäure, Stearinsäure, Nonadecansäure, Arachinsäure, Palmitoleinsäure, Ölsäure, Erucasäure, Linolsäure, Linolensäure, Elaeostearinsäure, Arachidonsäure, Fettsäuren aus der technischen Verseifung von natürlichen Ölen und Fetten wie beispielsweise Rapsöl, Sonnenblumenöl, Leinöl, Ölbaumöl, Kokosnussöl, Ölpalmkernöl und Ölpalmöl, sowie technische Gemische von Fettsäuren, welche solche Säuren enthalten. Als Carbonsäuren R⁵COOH geeignet sind zum anderen aromatische Carbonsäuren, beispielsweise Benzoesäure oder die stellungsisomeren Tolylsäuren, Ethyl- oder Isopropyl- oder tert.-Butyl- oder Methoxy- oder Nitrobenzoesäuren.

Bevorzugte Aldehyde der Formel (IV b) sind 2,2-Dimethyl-3-lauroyloxy-propanal, 2,2-Dimethyl-3-myristoyloxypropanal 2,2-Dimethyl-3-palmitoyloxy-propanal, 2,2-Dimethyl-3-stearoyloxy-propanal und 2,2-Dimethyl-3-benzoyloxy-propanal, sowie analoge Ester anderer 2,2-disubstituierter 3-Hydroxyaldehyde.

In einer besonders bevorzugten Ausführungsform ist R⁵ ausgewählt aus der Gruppe bestehend aus Phenyl und den C₁₁-, C₁₃-, C₁₅- und C₁₇-Alkylgruppen.

Besonders bevorzugt ist 2,2-Dimethyl-3-lauroyloxypropanal.

Als Amin **B** der Formel [H₂N]ₙ-E-XH eignen sich insbesondere
- aliphatische Hydroxyamine wie 2-Aminoethanol, 2-Methylaminoethanol, 1-Amino-2-propanol, 3-Amino-1-propanol, 4-Amino-1-butanol, 4-Amino-2-butanol, 2-Amino-2-methylpropanol, 5-Amino-1-pentanol, 6-Amino-1-hexanol, 7-Amino-1-heptanol, 8-Amino-1-octanol, 10-Amino-1-decanol, 12-Amino-1-dodecanol, 4-(2-Aminoethyl)-2-hydroxyethylbenzol, 3-Aminomethyl-3,5,5-trimethyl-cyclohexanol; eine primäre Aminogruppe tragende Derivate von Glykolen wie Diethylenglykol, Dipropylenglykol, Dibutylenglykol und höheren Oligomeren und Polymeren dieser Glykole, beispielsweise 2-(2-Aminoethoxy)-ethanol, Triethylenglykol-monoamin, α-(2-Hydroxymethyl-ethyl)-ω-(2-aminomethylethoxy)-poly(oxy(methyl-1,2-ethandiyl)); eine Hydroxylgruppe und eine oder mehrere primäre Aminogruppen tragende Derivate von polyalkoxylierten drei- oder höherwertigen Alkoholen oder von polyalkoxylierten Diaminen; Produkte aus der einfachen Cyanoethylierung und anschliessender Hydrierung von Glykolen, beispielsweise 3-(2-Hydroxyethoxy)-propylamin, 3-(2-(2-Hydroxyethoxy)-ethoxy)-propylamin, 3-(6-Hydroxyhexyloxy)-propylamin;
- aliphatische Mercaptoamine wie 2-Aminoethanthiol (Cysteamin), 3-Aminopropanthiol, 4-Amino-1-butanthiol, 6-Amino-1-hexanthiol, 8-Amino-1-octanthiol, 10-Amino-1-decanthiol, 12-Amino-1-dodecanthiol; Aminothiozucker wie 2-Amino-2-deoxy-6-thioglucose;
- zwei- oder mehrwertige aliphatische Amine, welche neben einer oder mehreren primären Aminogruppen eine sekundäre Aminogruppe tragen, wie N-Methyl-1,2-ethandiamin, N-Ethyl-1,2-ethandiamin, N-Butyl-1,2-ethandiamin, N-Hexyl-1,2-ethandiamin, N-(2-Ethylhexyl)-1,2-ethandiamin, N-Cyclohexyl-1,2-ethandiamin, 4-Aminomethyl-piperidin, 3-(4-Aminobutyl)-piperidin, N-Aminoethyl-piperazin, Diethylentriamin (DETA), Bis-hexamethylentriamin (BHMT); Di- und Triamine aus der Cyanoethylierung oder Cyanobutylierung von primären Mono- und Diaminen, beispielsweise N-Methyl-1,3-propandiamin, N-Ethyl-1,3-propandiamin, N-Butyl-1,3-propandiamin, N-Hexyl-1,3-propandiamin, N-(2-Ethylhexyl)-1,3-propandiamin, N-Dodecyl-1,3-propandiamin, N-Cyclohexyl-1,3-propandiamin,3-Methylamino-1-pentyl-amin,3-Ethylamino-1-pentylamin,3-Butylamino-1-pentylamin, 3-Hexylamino-1-pentylamin, 3-(2-Ethylhexyl)amino-1-pentylamin,3-Dodecylamino-1-pentyl-amin,3-Cyclohexylamino-1-pentylamin, Dipropylentriamin (DPTA), N3-(3-Aminopentyl)-1,3-pentandiamin, N5-(3-Aminopropyl)-2-methyl-1,5-pentandiamin, N5-(3-Amino-1-ethylpropyl)-2-methyl-1,5-pentandiamin,und Fettdiamine wie N-Cocoalkyl-1,3-propandiamin, N-Oleyl-1,3-propandiamin, N-Soya-alkyl-1,3-propandiamin, N-Talgalkyl-1,3-propandiamin oder N-(C₁₆₋₂₂-Alkyl)-1,3-propandiamin, wie sie beispielsweise unter dem Handelsnamen Duomeen^{®} von Akzo Nobel erhältlich sind; die Produkte aus der Michael-artigen Addition von aliphatischen primären Di- oder Polyaminen mit Acrylnitril, Malein- oder Fumarsäurediestern, Citraconsäurediestern, Acryl- und Methacrylsäureestern und Itaconsäurediestern, umgesetzt im Molverhältnis 1:1.

Bevorzugte aliphatische Hydroxy- und Mercaptoamine sind solche, in denen die primäre Aminogruppe von der Hydroxyl- beziehungsweise der Mercaptogruppe durch eine Kette von mindestens 5 Atomen, oder durch einen Ring, getrennt sind, wie beispielsweise in 5-Amino-1-pentanol, 6-Amino-1-hexanol, 7-Amino-1-heptanol, 8-Amino-1-octanol, 10-Amino-1-decanol, 12-Amino-1-dodecanol, 4-(2-Aminoethyl)-2-hydroxyethylbenzol, 3-Aminomethyl-3,5,5-trimethyl-cyclohexanol, 2-(2-Aminoethoxy)-ethanol, Triethylenglykol-monoamin, α-(2-Hydroxymethylethyl)-ω-(2-aminomethylethoxy)-poly(oxy(methyl-1,2-ethandiyl)), 3-(2-Hydroxyethoxy)-propylamin, 3-(2-(2-Hydroxyethoxy)-ethoxy)-propylamin, 3-(6-Hydroxyhexyloxy)-propylamin, 6-Amino-1-hexanthiol, 8-Amino-1-octanthiol, 10-Amino-1-decanthiol und 12-Amino-1-dodecanthiolHydroxy-hexyloxy)-propylamin.

Bevorzugt als Amin **B** der Formel [H₂N]ₙ-E-XH sind einerseits zwei-oder mehrwertige aliphatische Amine, welche neben einer oder mehreren primären Aminogruppen eine sekundäre Aminogruppe tragen, insbesondere N-Methyl-1,2-ethandiamin, N-Ethyl-1,2-ethandiamin, N-Cyclohexyl-1,2-ethan-diamin, N-Methyl-1,3-propandiamin, N-Ethyl-1,3-propandiamin, N-Butyl-1,3-propandiamin, N-Cyclohexyl-1,3-propandiamin, 4-Aminomethyl-piperidin, 3-(4-Aminobutyl)-piperidin, DETA, DPTA, BHMT und Fettdiamine wie N-Cocoalkyl-1,3-propandiamin, N-Oleyl-1,3-propandiamin, N-Soyaalkyl-1,3-propandiamin und N-Talgalkyl-1,3-propandiamin. Bevorzugt sind auch aliphatische Hydroxy-und Mercaptoamine, in denen die primäre Aminogruppe von der Hydroxybeziehungsweise der Mercaptogruppe durch eine Kette von mindestens 5 Atomen, oder durch einen Ring, getrennt sind, insbesondere 5-Amino-1-pentanol, 6-Amino-1-hexanol und höhere Homologe davon, 4-(2-Aminoethyl)-2-hydroxyethylbenzol, 3-Aminomethyl-3,5,5-trimethyl-cyclohexanol, 2-(2-Aminoethoxy)-ethanol, Triethylenglykol-monoamin und höhere Oligo- und Polymere davon, 3-(2-Hydroxyethoxy)-propylamin, 3-(2-(2-Hydroxyethoxy)-ethoxy)-propylamin sowie 3-(6-Hydroxyhexyloxy)-propylamin.

Meist bevorzugt ist das Amin **B** ausgewählt aus der Gruppe bestehend aus Bis-hexamethylentriamin (BHMT), N-Cyclohexyl-1,2-ethandiamin, N-Cyclohexyl-1,3-propandiamin, und 2-(2-Aminoethoxy)-ethanol.

Die Umsetzung zwischen einem Aldehyd **A** und einem Amin **B** führt dabei zu Hydroxyaldiminen, wenn als Amin **B** ein Hydroxyamin eingesetzt wird; zu Mercaptoaldiminen, wenn als Amin B ein Mercaptoamin eingesetzt wird; zu Aminoaldiminen, wenn als Amin **B** ein zwei- oder mehrwertiges Amin, welches neben einer oder mehreren primären Aminogruppen eine sekundäre Aminogruppen trägt, eingesetzt wird.

In einer Ausführungsform weisen die Aldimine der Formel (I) einen Substituenten N-R⁸ als Substituenten X auf. Derartige Aldimine der Formel (I) lassen sich dadurch herstellen, dass mindestens ein sterisch gehinderter aliphatischer Aldehyd **A** der Formel (IV) mit mindestens einem zwei- oder mehrwertigen aliphatischen primären Amin **C** der Formel [H₂N]ₙ-E-NH₂ in einem ersten Schritt zu einem Zwischenprodukt der Formel (VII) umgesetzt wird, welches neben einer oder mehreren Aldiminogruppen noch mindestens eine, bevorzugt eine, primäre Aminogruppe enthält, und dieses Zwischenprodukt anschliessend in einem zweiten Schritt in einer Additionsreaktion mit einem Michael-Akzeptor der Formel (VIII) in einem Verhältnis der Anzahl Doppelbindungen : Anzahl NH₂-Gruppen = 1:1 umgesetzt wird. Dabei entsteht ein Aminoaldimin, welches neben einer oder mehreren Aldiminogruppen noch mindestens eine, bevorzugt eine, sekundäre Aminogruppe enthält.

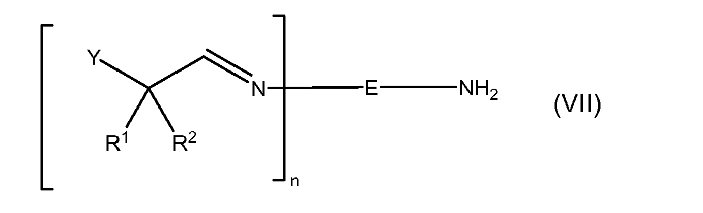

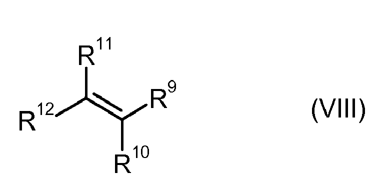

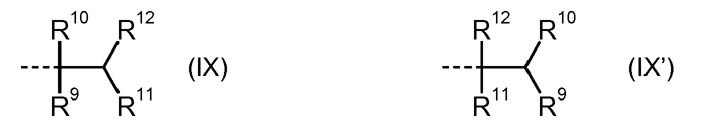

Somit entstehen Aldimine der Formel (I), bei welchen X für den Rest N-R⁸ steht, und R⁸ ein einwertiger Kohlenwasserstoffrest der Formel (IX) oder (IX') darstellt. In den Formeln (VIII), (IX) und (IX') stehen hierbei R⁹ für einen Rest, welcher ausgewählt ist aus der Gruppe bestehend aus -COOR¹³, -CN,
- NO₂, -PO(OR¹³)₂, -SO₂R¹³ und -SO₂OR¹³ und R¹⁰ für ein Wasserstoffatom oder einen Rest aus der Gruppe bestehend aus -R¹³, -COOR¹³ und
- CH₂COOR¹³ und R¹¹ und R¹² unabhängig voneinander für ein Wasserstoffatom oder einen Rest aus der Gruppe bestehend aus -R¹³,
- COOR¹³ und -CN, wobei R¹³ für einen einwertigen Kohlenwasserstoffrest mit 1 bis 20 C-Atomen steht.

Das Amin **C** ist ein aliphatisches Amin mit mindestens zwei primären Aminogruppen. Der Begriff "aliphatisches primäres Amin" bezeichnet ein aliphatisches Amin, in welchem die Aminogruppe eine primäre Aminogruppe ist.

Beispiele für geeignete Amine **C** der Formel [H₂N]ₙ-E-NH₂ sind aliphatische Polyamine wie Ethylendiamin, 1,2- und 1,3-Propandiamin, 2-Methyl-1,2-propandiamin, 2,2-Dimethyl-1,3-propandiamin, 1,3- und 1,4-Butandiamin, 1,3- und 1,5-Pentandiamin, 2-Butyl-2-ethyl-1,5-pentandiamin, 1,6-Hexamethylendiamin (HMDA), 2,2,4- und 2,4,4-Trimethylhexamethylendiamin und Mischungen davon (TMD), 1,7-Heptandiamin, 1,8-Octandiamin, 2,4-Dimethyl-1,8-octandiamin, 4-Aminomethyl-1,8-octandiamin, 1,9-Nonandiamin, 2-Methyl-1,9-nonandiamin, 5-Methyl-1,9-nonandiamin, 1,10-Decandiamin, Isodecandiamin, 1,11-Undecandiamin, 1,12-Dodecandiamin, Methyl-bis-(3-amino-propyl)amin, 1,5-Diamino-2-methylpentan (MPMD), 1,3-Diaminopentan(DAMP), 2,5-Dimethyl-1,6-hexamethylendiamin, cycloaliphatische Polyamine wie 1,2-, 1,3- und 1,4-Diaminocyclohexan, Bis-(4-aminocyclohexyl)-methan (H₁₂MDA), Bis-(4-amino-3-methylcyclohexyl)-methan, Bis-(4-amino-3-ethylcyclohexyl)-methan, Bis-(4-amino-3,5-dimethylcyclohexyl)-methan, Bis-(4-amino-3-ethyl-5-methylcyclohexyl)-methan (M-MECA), 1-Amino-3-aminomethyl-3,5,5-trimethylcyclohexan (= Isophorondiamin oder IPDA), 2- und 4-Methyl-1,3-diaminocyclohexan und Mischungen davon, 1,3- und 1,4-Bis-(aminomethyl)cyclohexan, 1,3,5-Tris-(aminomethyl)cyclohexan, 1-Cyclohexylamino-3-aminopropan, 2,5(2,6)-Bis-(aminomethyl)-bicyclo[2.2.1]heptan (NBDA, hergestellt von Mitsui Chemicals), 3(4),8(9)-Bis-(aminomethyl)-tricyclo[5.2.1.0^{2,6}]decan, 1,4-Diamino-2,2,6-trimethylcyclohexan (TMCDA), 3,9-Bis-(3-aminopropyl)-2,4,8,10-tetraoxa-spiro[5.5]undecan, arylaliphatische Polyamine wie 1,3-Xylylendiamin (MXDA), 1,4-Xylylendiamin (PXDA), 1,3,5-Tris-(aminomethyl)benzol, Ethergruppenhaltige aliphatische Polyamine wie Bis-(2-aminoethyl)ether, 4,7-Dioxadecan-1,10-diamin, 4,9-Dioxadodecan-1,12-diamin und höhere Oligomere davon, Polyoxyalkylen-Polyamine mit theoretisch zwei oder drei Aminogruppen, erhältlich beispielsweise unter dem Namen Jeffamine^{®} (hergestellt von Huntsman Chemicals). Bevorzugt sind Di- oder Triamine, in denen die primären Aminogruppen durch eine Kette von mindestens 5 Atomen, oder durch einen Ring, getrennt sind, insbesondere 1,5-Diamino-2-methylpentan, 1,6-Hexamethylendiamin, 2,2,4- und 2,4,4-Trimethylhexamethylendiamin und Mischungen davon, 1,10-Decandiamin, 1,12-Dodecandiamin, 1,3- und 1,4-Diaminocyclohexan, Bis-(4-aminocyclohexyl)-methan, Bis-(4-amino-3-methylcyclohexyl)-methan, 1-Amino-3-aminomethyl-3,5,5-trimethylcyclohexan, 1,3- und 1,4-Bis-(aminomethyl)cyclohexan, 2,5(2,6)-Bis-(aminomethyl)-bicyclo[2.2.1]heptan, 3(4),8(9)-Bis-(aminomethyl)-tricyclo[5.2.1.0^{2,6}]decan, 1,4-Diamino-2,2,6-trimethylcyclohexan (TMCDA), 3,9-Bis-(3-aminopropyl)-2,4,8,10-tetraoxaspiro[5.5]undecan, 1,3- und 1,4-Xylylendiamin, 1,3,5-Tris-(aminomethyl)benzol sowie Polyoxyalkylen-Polyamine mit theoretisch zwei oder drei Aminogruppen, erhältlich beispielsweise unter dem Namen Jeffamine^{®} (hergestellt von Huntsman Chemicals).

Beispiele für geeignete Michael-Akzeptoren der Formel (VIII) sind Malein- oder Fumarsäurediester wie Dimethylmaleinat, Diethylmaleinat, Dibutylmaleinat, Diethylfumarat; Citraconsäurediester wie Dimethylcitraconat; Acryl- oder Methacrylsäureester wie Methyl(meth)acrylat, Ethyl(meth)acrylat, Butyl(meth)acrylat, Lauryl(meth)acrylat, Stearyl(meth)acrylat, Tetrahydrofuryl-(meth)acrylat, Isobornyl(meth)acrylat, Itaconsäurediester wie Dimethylitaconat; Zimtsäureester wie Methylcinnamat; Vinylphosphonsäurediester wie Vinylphosphonsäuredimethylester; Vinylsulfonsäureester, insbesondere Vinylsulfonsäurearylester; Vinylsulfone; Vinylnitrile wie Acrylnitril, 2-Pentennitril oder Fumaronitril; 1-Nitroethylene wie β-Nitrostyrol, und Knoevenagel-Kondensationsprodukte, wie beispielsweise solche aus Malonsäurediestern und Aldehyden wie Formaldehyd, Acetaldehyd oder Benzaldehyd. Bevorzugt sind Maleinsäurediester, Acrylsäureester, Phosphonsäurediester und Vinylnitrile.

Die Umsetzung des Aldehyds A mit dem Amin C zum Zwischenprodukt der Formel (VII) erfolgt in einer Kondensationsreaktion unter Abspaltung von Wasser, wie sie weiter oben für die Umsetzung des Aldehyds A mit dem Amin B beschrieben wird. Die Stöchiometrie zwischen dem Aldehyd A und dem Amin C wird dabei so gewählt, dass pro mol Aldehyd A mindestens 1 mol Amin C eingesetzt werden. Es wird ein lösemittelfreies Herstellverfahren bevorzugt, wobei das bei der Kondensation gebildete Wasser mittels Anlegen von Vakuum aus der Reaktionsmischung entfernt wird.

Die Umsetzung des Zwischenprodukts der Formel (VII) mit dem Michael-Akzeptor der Formel (VIII) erfolgt beispielsweise dadurch, dass das Zwischenprodukt mit einer stöchiometrischen oder leicht überstöchiometrischen Menge des Michael-Akzeptors der Formel (VIII) gemischt wird und die Mischung bei Temperaturen von 20 bis 110°C bis zum vollständigen Umsatz des Zwischenprodukts zum Aldimin der Formel (I) erwärmt wird. Die Umsetzung erfolgt bevorzugt ohne eine Verwendung von Lösemitteln.

R³ steht bevorzugt für H.

Der Substituent E weist bevorzugt 3 bis 6 C-Arome auf.

Bevorzugte Aldimine der Formel (I) sind geruchsarm. Besonders bevorzugte Aldimine der Formel (I) sind geruchsfrei.

In einer bevorzugten Ausführungsform steht Y¹ für den Rest der Formel (III a) und R⁴ für einen linearen oder verzweigten Alkylrest mit 6 bis 30, insbesondere mit 11 bis 30, C-Atomen.

In einer besonders bevorzugten Ausführungsform steht Y¹ für den Rest der Formel (III b) und R⁵ für einen linearen oder verzweigten Alkylrest mit 6 bis 30, insbesondere mit 11 bis 30, C-Atomen.

Besonders bevorzugte Aldimine der Formel (I) weisen die Struktur der Formel (III b-1) oder (III b-2) oder (III b-3) oder (III b-4) auf.

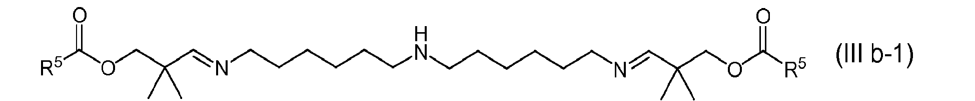

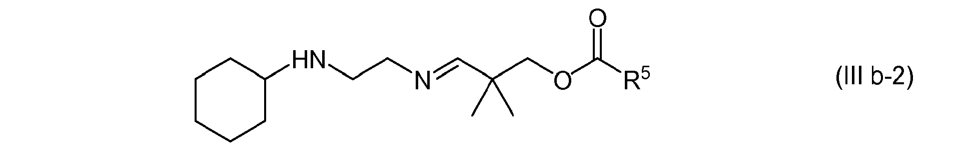

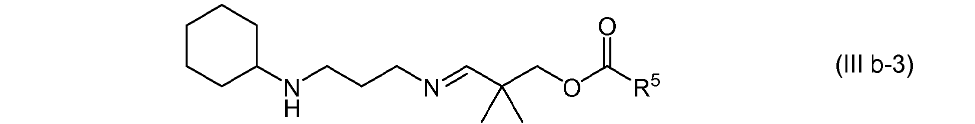

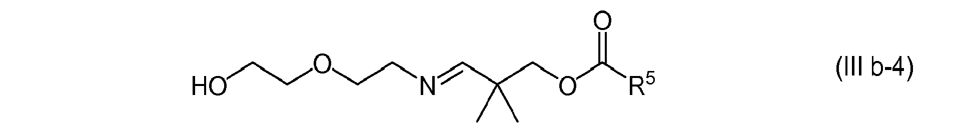

Das vorgängig beschriebene Aldimin der Formel (I) kann alleine als Reinigungsmittel oder als Bestandteil einer Reinigungsmittelzusammensetzung für reaktive Polyurethanzusammensetzungen eingesetzt werden.

Reaktive Polyurethanzusammensetzungen weisen reaktive NCO-Gruppen auf. Die XH-Gruppe reagiert mit den Isocyanatgruppen der reaktiven Polyurethanzusammensetzungen, d.h. die NCO Gruppen werden blockiert, so dass die so blockierten Polyurethanzusammensetzungen keine freien NCO-Gruppen aufweisen.

Es ist in diesem Zusammenhang wichtig zu betonen, dass es wesentlich für die vorliegende Erfindung ist, dass das Aldimin der Formel (I) lediglich eine Gruppe XH aufweisen. Wären nämlich mehrere XH-Gruppen vorhanden, würde bei einer derartigen Reaktion mit den Isocyanatgruppen eine Vernetzung erfolgen und eine Reinigung wäre nicht mehr möglich, sondern es würde eine ähnliche Art von Verklebung en oder Anhärtungen erfolgen, wie sie bei der Reaktion der reaktiven Polyurethanzusammensetzungen (ohne Benutzung einer Reinigerzusammensetzung) im Kontakt mit Feuchtigkeit gebildet werden.

Infolge dieser Blockierungsreaktion der den Isocyanatgruppen, insbesondere der Isocyanatgruppen aufweisenden Polyurethanpolymeren, der reaktiven Polyurethanzusammensetzungen, durch das Aldimin der Formel (I) wiesen die so blockierten Polyurethanzusammensetzungen Aldimingruppen auf.

Somit stellt eine Reinigungsmittelzusammensetzung für reaktive Polyurethanzusammensetzungen, insbesondere für reaktive Polyurethanheissschmelzklebstoffe einen weiteren Aspekt der vorliegenden Erfindung dar.

Eine derartige Reinigungsmittelzusammensetzung enthält insbesondere
- mindestens ein Aldimin der Formel (I), wie es vorgängig beschrieben wurde;
   sowie
- mindestens ein Trägermittel **A**, welches keine gegenüber Isocyanat reaktive funktionellen Gruppen trägt.

Unter einem "Trägermittel" im Sinne dieser Schrift werden Polymere verstanden, welche thermoplastisch sind und die Verbindung **B** im Wesentlichen homogen aufnehmen können.

Es ist wichtig, dass das Trägermittel **A** keine Isocyanat-reaktive Gruppen aufweist, um zu verhindern, dass das Trägermittel **A** bei Kontakt mit der reaktiven Polyurethanzusammensetzung vernetzend wirkt.

Als Trägermittel **A** kommen verschiedenste Materialien in Frage, jedoch muss das Trägermittel **A** thermoplastisch sein und darf keine Isocyanat-reaktive Gruppen aufweisen. Weiterhin sollte vorteilhaft das Trägermittel **A** bei der Applikationstemperatur eine mit dem zu verdrängenden reaktiven Polyurethan-Heissschmelzklebstoff vergleichbare Viskosität aufweisen.

Ein geeignetes nicht reaktives Trägermittel **A** ist insbesondere ein blockiertes Polyurethanprepolymer, ein Kohlenwasserstoffharz, ein blockierter Polyester, ein blockiertes Polyol oder ein Copolymer aus mindestens einem der Monomere, welche ausgewählt sind aus der Gruppe bestehend aus Ethylen, Propylen, Butylen, Butadien, Vinylester, Vinylchlorid, Styrol, Ester der Acrylsäure und Ester der Methacrylsäure.

Als nicht reaktive Polyurethane kommen beispielsweise stöchiometrisch blockierte Polyurethanprepolymere in Frage, welche mit einem Blockierungsmittel inertisiert sind.

Einerseits sind Isocyanatgruppen aufweisende Polyurethanprepolymere mit Blockierungsmittel wie beispielsweise ε-Caprolactam oder monofunktionellen Alkoholen oder Aminen oder Mercaptanen blockierbar. Andererseits können OH-Gruppen aufweisende Polyurethanprepolymere durch monofunktionelle Isocyanate, insbesondere p-Tosylisocyanat blockiert werden.

In einer bevorzugten Ausführungsform wird ein nicht reaktives Polyurethan eingesetzt, welches aus dem zu verdrängenden reaktiven Polyurethan-Heissschmelzklebstoff besteht, dessen reaktive Isocyanatgruppen mit einem monofunktionellen Alkohol, Amin, Mercaptan, Amid oder Imid, blockiert worden sind.

In einer besonders geeigneten Ausführungsform ist das Trägermittel das Isocyanatgruppen-freie Umsetzungsprodukt des vorgängig beschriebenen Aldehyds der Formel (I) und eines Isocyanatgruppen aufweisenden Polyurethanpolymeren, insbesondere dasjenige der zu reinigenden reaktiven Polyurethanzusammensetzung. Ein derartiges Isocyanatgruppen-freie Umsetzungsprodukt wird vorzugsweise mit einem stöchiometrischen OH/NCO-Überschuss an Aldehyd der Formel (I) durchgeführt. Somit ist es vorteilhaft, wenn noch eine gewisse Restmenge an nicht umgesetzten Aldehyd der Formel (I) vorhanden bleibt.

Als blockierte Polyole kommen insbesondere im Wesentlichen total veretherte Polyole in Frage. Das Polyolgerüst dieses veretherten Polyols ist insbesondere ausgewählt aus der Gruppe umfassend Polyetherpolyole, Polyesterpolyole, Polycaprolactonpolyole. Im Besonderen handelt es sich hierbei um Dialkoxypolyalkoxydiole, bevorzugt Dimethoxypolyethylenglycole oder Dimethoxypolypropylenglycole, oder Alkoxy-Polyesterpolyole. Die veretherten Polyesterpolyole weisen insbesondere weder freie Hydroxygruppen noch frei Carboxylgruppen auf.

Andererseits sind als blockierte Polyole geeignet vor allem Polyesterpolyole, Polycarbonatpolyole, Polyhydroxyterminierte Polybutadienpolyole, Polyetherpolyole, insbesondere Polyoxyalkylenpolyole, welche mit einer monofunktionellen Isocyanatverbindung stöchiometrisch blockiert werden, so dass weder freie Hydroxygruppen noch frei Carboxylgruppen vorhanden sind.

Bei den erwähnten blockierten Trägermitteln ist darauf zu achten, dass das Blockierungsmittel bei der Applikationstemperatur der Reinigungsmittelzusammensetzung nicht zu wesentlichen Anteilen deblockiert.

Weiterhin geeignet als Trägermittel **A** sind Kolophonium, Kohlenwasserstoffharze, hydrierte oder nicht hydrierte Terpenharze sowie Petroleumharze.

Geeignete Homo- oder Copolymere sind thermoplastische Homo- oder Copolymere, insbesondere diejenigen welche aus der Polymerisation von Monomere, welche C=C-Doppelbindungen aufweisen hergestellt werden, insbesondere mindestens einem Monomer ausgewählt aus der Gruppe umfassend Ethylen, Propylen, Butadien, Vinylester, Vinylchlorid, Styrol sowie Ester der Acryl- oder Methacrylsäure. Bevorzugt sind Copolymere aus Ethylen und Vinylester, insbesondere Ethylen/Vinylacetat-Copolymere (EVA).

Als besonders geeignet haben sich Ethylen/Vinylacetat-Copolymere als Trägermittel gezeigt. Im Vergleich zu den blockierten Polyurethanprepolymeren zeichnen sich die Ethylen/Vinylacetat-Copolymere vor allem durch einen tiefen Preis und breite kommerzielle Erhältlichkeit aus. Entgegen der Fachmeinung, wie sie beispielsweise in EP 0 544 058 A2 geäussert wird, wurde gefunden, dass EVA sehr gut mit Polyurethanklebstoffen mischbar ist und deshalb für die Anwendung als Trägermaterial einer Reinigungsmittelzusammensetzung geeignet ist.

Das Trägermittel **A** weist vorteilhaft eine Glasübergangstemperatur (Tg) auf, die tiefer als die Anwendungstemperatur der Reinigungsmittelzusammensetzung ist.

Das Trägermittel **A** wird insbesondere derart ausgewählt, dass die Polarität des Trägermittels **A** auf diejenige des zu verdrängenden Heissschmelzklebstoffes abgestimmt ist. Weil Polyurethanheissschmelzklebstoffe vorzugsweise polaren Charakter aufweisen sollten, bedeutet dies, dass vor allem polare Trägermittel **A** bevorzugt werden.

Je nach Anteil des Trägermittels **A** in der Reinigungsmittelzusammensetzung und der der für die Reinigung eingesetzten Menge Reinigungsmittelzusammensetzung kann es jedoch unter Umständen aufgrund des Trägermittels **A** nötig werden, einen geringen Anteil der Mischfraktionen zu entsorgen.

Vor allem in diesen Fällen ist es vorteilhaft, wenn die Reinigungsmittelzusammensetzung weiterhin einen Farbstoff oder ein Pigment umfasst. Bevorzugt werden Farbstoffe eingesetzt, die gelöst im Trägermittel **A** vorliegen. Ein derartiger Farbstoff ermöglicht es visuell besser zu beurteilen ob und wie viel von der Mischfraktion entsorgt werden muss.

Weiterhin ist es vorteilhaft, wenn die Reinigungsmittelzusammensetzung weiterhin einen Farbstoff oder ein Pigment enthält, um visuell einfach festzustellen, wenn der Vorgang der Blockierung der reaktive Polyurethanzusammensetzung durch die Reinigungsmittelzusammensetzung abgeschlossen ist.

Um möglichst die Vorteile der vorliegenden Erfindung nutzen zu können, nämlich dass möglichst wenig Material entsorgt werden muss, ist es deshalb erwünscht, dass der Anteil des Trägermittels **A** nicht allzu hoch ausfällt. Es hat sich gezeigt, dass der Anteil des Trägermittels **A** vorteilhaft nicht mehr als 50 Gewichts-%, insbesondere zwischen 5 und 30 Gewichts-%, bezogen auf die Reinigungsmittelzusammensetzung, beträgt.

Speziell vorteilhaft ist es deshalb, wenn lediglich das Aldimin der Formel allein als Reinigungsmittel benutzt wird.

Es hat sich weiterhin gezeigt, dass es vorteilhaft ist, wenn die Reinigungsmittelzusammensetzung weiterhin mindestens einen Katalysator für die Reaktion der Gruppe HX mit Isocyanat, insbesondere eine Organozinnverbindung und/oder eine Bismutverbindungen und/oder ein tertiäres Amin, enthält.

Derartige Organozinnverbindungen sind dem Fachmann bestens bekannt, beispielsweise Dibutylzinndiacetat, Dibutylzinndilaurat, Dibutylzinndichlorid, Dibutylzinndiacetylacetonat oder Dioctylzinndilaurat.

Derartige Bismutverbindungen sind dem Fachmann bekannt, beispielsweise Bismuttrioctoat oder Bismuttris(neodecanoat).

Derartige als Katalysatoren einsetzbare tertiäre Amine sind insbesondere 2,2'-Dimorpholinodiethylether und 1,4-Diazabicyclo[2.2.2]octan.

Die Reinigungsmittelzusammensetzung kann bei Bedarf weiterhin Füllstoffe enthalten. Um eine Schädigung der Anlagen und Geräte zu vermeiden, sollten in der Regel diese Füllstoff jedoch nicht abrasiv sein. Es sind Reinigungsmittelzusammensetzungen bevorzugt, die keine Füllstoffe enthalten.

Die Reinigungsmittelzusammensetzung kann bei Bedarf weitere Bestandteile enthalten, wie Weichmacher, Stabilisatoren, insbesondere Hitzestabilisatoren, oberflächenaktive Hilfsmittel, wie beispielsweise Tenside, wie sie in konventionellen Reinigungsmittel vorkommen.

Bei der Verwendung von Weichmachern sind jedoch Grenzen gesetzt. Es ist bei hohen Gehalten oder gewissen Typen der Nachteil vorhanden, dass durch Migrationseffekte eine Weichmacher enthaltende Reinigungsmittelzusammensetzung bei Lagerung schmierig wird und deshalb schwierig hand zu haben wird.

Die optionalen Bestandteile der Reinigungsmittelzusammensetzung sollen derart ausgewählt werden, dass sie keine funktionellen Gruppen aufweisen, die mit Isocyanaten, insbesondere bei der Reinigungstemperatur, nicht reagieren.

Das vorgängig beschriebene Aldimin der Formel (I) oder die vorgängig beschriebene Reinigungsmittelzusammensetzung eignet sich speziell gut zum Reinigen von Herstell- und Verarbeitungsanlagen von reaktiven Polyurethanzusammensetzungen, insbesondere von reaktiven Polyurethan-Heissschmelzklebstoffen

Einen weiteren Aspekt der vorliegenden Erfindung betrifft deshalb ein Verfahren zur Reinigung einer Herstell- oder Applikationsanlage oder eines Applikationsgerätes von reaktiven Polyurethanzusammensetzungen.

In diesem Verfahren wird ein vorgängig beschriebenes Aldimin der Formel (I) oder eine Reinigungsmittelzusammensetzung, wie sie vorgängig beschrieben wurde, zu einer in dieser Anlage oder Gerät befindlichen reaktiven Polyurethanzusammensetzung zugegeben und diese aus der Anlage oder dem Gerät verdrängt.

Grundsätzlich kann die Erfindung jede reaktive Polyurethanzusammensetzung betreffen, solange als, dass sie Isocyanatgruppen aufweist.

Diese reaktiven Polyurethanzusammensetzungen sind insbesondere als Klebstoffe oder Dichtstoffe oder Gussharz oder Beschichtung verwendbar.

Insbesondere sind als einkomponentige reaktive Polyurethanzusammensetzungen basierend auf Isocyanatgruppen aufweisende Polyurethanpolymeren. Derartige Isocyanatgruppen aufweisende Polyurethanpolymere werden in bekannter Art und Weise aus Polyolen und Polyisocyanaten hergestellt.

Wichtig ist die vorliegende Erfindung einerseits für einkomponentige reaktive Polyurethan-Dichtstoffe oder -Klebstoffe basierend auf Isocyanat-gruppen aufweisende Polyurethanpolymeren, welche bei Raumtemperatur appliziert werden und mit Luftfeuchtigkeit aushärten. Derartige Dichtstoffe oder Klebstoffe sind beispielsweise unter den Handelsmarken Sikaflex® oder SikaTack® kommerziell von Sika Schweiz AG erhältlich.

Besonders wichtig ist die vorliegende Erfindung andererseits für einkomponentige reaktive Polyurethan-Klebstoffe basierend auf Isocyanatgruppen aufweisende Polyurethanpolymeren, welche bei höherer Temperatur appliziert werden und mit Luftfeuchtigkeit aushärten. Derartige Klebstoffe werden auch als Warmmelt-Polyurethanklebstoffe (Applikationstemperatur 50° bis 80°C) oder reaktive Polyurethan-Heissschmelzklebstoffe (reactive polyurethane hotmelts) (Applikationstemperatur grösser als 80°C) genannt.

Reaktive Polyurethan-Heissschmelzklebstoffe (reactive polyurethane hotmelts) sind hierbei die meist bevorzugten reaktive Polyurethanzusammensetzungen. Derartige reaktive Polyurethan-Heissschmelzklebstoffe sind beispielsweise von Sika Automotive GmbH, Deutschland, unter dem Handelsnamen SikaMelt® kommerziell erhältlich.

Das Aldehyd der Formel (I), beziehungsweise die Reinigungsmittelzusammensetzung, muss zumindest für die Zeit der Reinigung bei Temperaturen stabil sein, denen sie während der Reinigung ausgesetzt ist. Es ist anstrebenswert, der Aldehyd bzw. die Reinigungsmittelzusammensetzung mindestens einige Stunden, insbesondere Tage, bei der Reinigungstemperatur verweilen kann, ohne dass der Aldehyd bzw. die Reinigungsmittelzusammensetzung zu wesentlichen Anteilen zersetzt oder abgebaut wird.

Der Aldehyd bzw. die Reinigungsmittelzusammensetzung ist vorteilhaft möglichst frei von Wasser oder Feuchtigkeit. Es sollte deshalb bei der Herstellung möglichst trockene oder getrocknete Rohstoffe verwendet werden und auf aus der Umgebung wasseraufnehmende Inhaltsstoffe verzichtet werden.

Ein solches Reinigungsmittel ist äusserst effizient, kostengünstig und ökologisch und arbeitshygienisch äusserst vorteilhaft.

Am Ende der Herstellung kann die Reinigungsmittelzusammensetzung in die richtige Darreichungsform gebracht, wie sie für die Anwendung benötigt wird. Für die Reinigung von Heissschmelzklebstoffen ist eine Darreichungsform in fester Form vorteilhaft. Sie kann beispielsweise als Granulat, in Schuppenform oder als Block vorliegen. Diese Überführung in die Darreichungsform erfolgt in bekannter Weise und kann unmittelbar aus der Schmelze oder während oder nach dem Abkühlung erfolgen. Vorteilhaft wird die Reinigungsmittelzusammensetzung direkt nach Herstellung aus der Schmelze in die Anwendungsform überführt. Bei der Auswahl dieser Darreichungsform ist es vorteilhaft eine solche Form zu wählen, die eine Dosierung der Reinigungsmittelzusammensetzung auch noch nach längerer Lagerzeit ermöglicht.

Ebenso ist darauf zu achten, dass die für das Aldehyd bzw. die Reinigungsmittelzusammensetzung verwendete Verpackung einen adäquaten Schutz bietet und insbesondere feuchtigkeitsundurchlässig sein sollte.

Die Zugabe in die zu reinigende Anlage oder das zu reinigende Gerät sollte vor allem bei Heissschmelzklebstoffen in fester Form oder in Form einer Schmelze erfolgen. Es ist bevorzugt, wenn die Reinigungsmittelzusammensetzung in Granulat oder Schuppenform vorliegt. Die Reinigungstemperatur ist die üblicherweise die Verarbeitungstemperatur des reaktiven Polyurethan-Heissschmelzklebstoffes, typischerweise 80 bis 180°C, insbesondere 90 bis 150°C. Es kann jedoch vorteilhaft sein, die Reinigung bei höherer Temperatur durchzuführen, damit der zu verdrängende reaktive Polyurethan-Heissschmelzklebstoff nieder viskoser wird. Durch die Zersetzungstemperatur des Polyurethan-Heissschmelzklebstoffes oder der Reinigungsmittelzusammensetzung sind hier jedoch systembedingte Obergrenzen vorhanden, die unter keinen Umständen überschritten werden dürfen.

Bei der Reinigung von reaktiven Polyurethanklebstoffen oder -Dichtstoffen, welche bei Raumtemperatur appliziert werden, erfolgt die Zugabe und Reinigung vorteilhaft bei Raumtemperatur.

Eine Reinigung einer Anlage oder eines Gerätes erfolgt typischerweise wie folgt für eine Reinigungsmittelzusammensetzung beschrieben. Es versteht sich von selbst, dass das entsprechende auch für eine Reinigung mittels eines Aldehydes der Formel (I) gilt:
Die Zuführung einer reaktiven Polyurethanzusammensetzung wird unterbrochen und die Reinigungsmittelzusammensetzung in die Anlage oder das Gerät zugegeben. Anschliessend wurde während dem Fördern mit der Förderung der Polyurethanzusammensetzung weitergefahren, bis gewährleistet ist, dass am Austritt der Anlage oder dem Gerät keine reaktive Polyurethanzusammensetzung mehr vorhanden ist. Dieser Zeitpunkt nach Zugabe der Reinigungsmittelzusammensetzung hängt von vielen Faktoren ab, insbesondere von der Reinigungstemperatur, dem Gehalt an freien Isocyanat-gruppen der reaktive Polyurethanzusammensetzung, die Dimensionen und Geometrie der zu reinigenden Anlage oder Gerätes sowie der Konzentration des Aldehyds der Formel (I) in der Reinigungsmittelzusammensetzung.

Dieser Zeitpunkt kann entweder visuell bestimmt werden, wenn die Reinigungsmittelzusammensetzung einen entsprechend gewählten Farbstoff oder Pigment enthält. Andererseits kann dieser Zeitpunkt durch Infrarot-Absorptions- bzw. Transmissionsspektroskopie bestimmt werden, nämlich, dann wenn die für die NCO-Gruppe charakteristische IR-Bande (2270 cm⁻¹) verschwunden ist. Es ist jedoch auch durchaus möglich, dass bei einer Anlage und der jeweiligen spezifischen reaktiven Polyurethanzusammensetzung in Kombination mit fixierten Anlagenparametern eine früher für diese Kombination bestimmte Zeit für das verlässliche Blockierung verwendet werden kann.

Die so gereinigte Anlage oder das so gereinigte Gerätes kann über einen längeren Zeitpunkt problemlos unbenutzt gelassen werden.

Beim Wiederbeschicken der Anlage oder des Gerätes mit (neuer) reaktiver Polyurethanzusammensetzung kann einfach weitergearbeitet werden. Es ist hierbei lediglich zu beachten, dass am Anfang nach dem Anfahren der Anlage eine kleine Menge weggenommen werden muss, bis gewährleistet ist, dass eine genügend grosse Menge an nicht-blockierten reaktiven Polyurethanzusammensetzung vorhanden ist. Es hat sich als Erfahrungswert gezeigt, dass eine verlässlich benutzbare Mischfraktion maximal 30 Gew.-% an mit Aldimin der Formel (I) blockiertem Polyurethanpolymeren aufweisen sollte. Es ist jedoch ein grosser Vorteil der vorliegenden Erfindung, dass diese weggenommene Menge der reaktive Polyurethanzusammensetzung vor der Zuführung zur Anlage problemlos zugemischt werden können, so dass faktisch dank dieser Recylierung kein Abfall entsteht.

Der Aldehyd der Formel (I) und die Reinigungsmittelzusammensetzung eignet sich speziell für das Reinigen von Verarbeitungsanlagen sowie von Applikationsgeräten, insbesondere von Auftragsapparaturen, von reaktiven Polyurethanzusammensetzungen, insbesondere von reaktiven Polyurethan-Heissschmelzklebstoffen. Typischerweise weisen diese Anlagen und Geräte sich verengende Teile wie Schlitze oder Düsen, bewegliche Teile wie Schnecken, Walzen, Rührer, Flügel, Pumpen oder auch Schläuche und Rohre auf, welche manuell nur mit sehr grossem Arbeits- und Kostenaufwand zu reinigen sind. Mit dem erfindungsgemässen Aldehydes der Formel (I) bzw. Reinigungsmittelzusammensetzung kann eine gründliche und trotzdem schonende und schnelle Reinigung dieser Anlagen und Geräte erfolgen, indem die reaktive Polyurethanzusammensetzung hieraus entfernt wird.

Es versteht sich von selbst, dass solche Reinigungsmittelzusammensetzungen auch für das Reinigen von Anlagen verwendet werden können, in welchen reaktive Polyurethanzusammensetzungen hergestellt werden.

### Beispiele

Die Beispiele dienen zur Illustration der Erfindung und sind keineswegs als die Erfindung beschränkend aufzufassen.

### Beschreibung der Messmethoden

Die **Infrarotspektren** wurden auf einem FT-IR Gerät 1600 von Perkin-Elmer gemessen (horizontale ATR-Messeinheit mit ZnSe-Kristall), die Proben wurden unverdünnt als Filme aufgetragen. Die Absorptionsbanden sind in Wellenzahlen (cm⁻¹) angegeben (Messfenster: 4000-650 cm⁻¹).

**¹H-NMR-Spektren** wurden gemessen auf einem Spektrometer des Typs Bruker DPX-300 bei 300.13 MHz; die chemischen Verschiebungen δ sind angegeben in ppm relativ zu Tetramethylsilan (TMS), Kopplungskonstanten J sind angegeben in Hz. Die Kopplungsmuster (t, m) wurden angegeben, auch wenn es sich nur um Pseudokopplungsmuster handelt.

Die **Viskosität** wurde auf einem thermostatisierten Kegel-Platten-Viskosimeter Physica UM (Kegeldurchmesser 20 mm, Kegelwinkel 1°, Kegelspitze-Platten-Abstand 0.05 mm, Schergeschwindigkeit 10 bis 1000 s⁻¹) gemessen.

Der totale **Gehalt an Aldiminogruppen und freien Aminogruppen** in den hergestellten Verbindungen ("Amin-Gehalt") wurde titrimetrisch bestimmt (mit 0.1N HClO₄ in Eisessig, gegen Kristallviolett) und ist stets angegeben in mmol NH₂/g (auch wenn es sich nicht nur um primäre Aminogruppen handelt).

### Herstellung beispielhafter Aldehyde

### Aldimin AL1

In einem Rundkolben wurden unter Stickstoffatmosphäre 30.13 g (0.106 mol) 2,2-Dimethyl-3-lauroyloxy-propanal vorgelegt. Unter kräftigem Rühren wurden aus einem Eintropftrichter innerhalb von 5 Minuten 15.00 g (0.096 mol) N-Cyclohexyl-1,3-propandiamin zugegeben, wobei die Temperatur des Reaktionsgemisches auf 36°C anstieg. Danach wurden die flüchtigen Bestandteile im Vakuum entfernt (10 mbar, 80°C). Man erhielt 43.2 g einer farblosen, bei Raumtemperatur dünnflüssigen, klaren und geruchlosen Flüssigkeit, die einen Amin-Gehalt von 4.39 mmol NH₂/g aufwies. Das Produkt liegt grösstenteils in der offenkettigen (Aldimin-) Form vor.

IR: 3308 (N-H), 2921, 2851, 2659, 1737 (C=O), 1668 (C=N), 1465, 1449, 1418sh, 1393,1366, 1346, 1301, 1248, 1158, 1111, 1068, 1020, 1002, 938, 888, 845, 797, 721. ¹H-NMR (CDCl₃, 300 K): δ 7.53 (s, 1 H, CH=N), 4.01 (s, 2 H, CH₂O), 3.43 (t, 2 H, CH=NC*H*₂CH₂), 2.65 (*t*, 2 H, NHC*H*₂), 2.40 (s, 1 H, Cy-C¹*H*NH), 2.29 (*t*, 2 H, CH₂CO), 1.86 (m, 2 H, 2 Cy-H), 1.72 (m, 4 H, 2 Cy-H und CH=NCH₂C*H*₂), 1.60 (m, 3 H, C*H*₂CH₂CO und CH₃N*H*CH₂), 1.26 (m, 22 H, CH₃-(C*H*₂)₈-CH₂CH₂CO und 6 Cy-H), 1.09 (s, 6 H, C(C*H*₃)₂-CH₂O), 0.88 (*t*, 3 H, C*H*₃-(CH₂)₁₀-CO).

### Aldimin AL2

In einem Rundkolben wurden unter Stickstoffatmosphäre 34.15 g (0.120 mol) 2,2-Dimethyl-3-lauroyloxy-propanal vorgelegt. Unter kräftigem Rühren wurden aus einem Eintropftrichter innerhalb von 5 Minuten 12.02 g (0.056 mol) Bis-hexamethylentriamin (BHMT-HP; Invista) zugegeben, wobei die Temperatur des Reaktionsgemisches auf 35°C anstieg. Danach wurden die flüchtigen Bestandteile im Vakuum entfernt (10 mbar, 80°C). Man erhielt 43.6 g einer farblosen, bei Raumtemperatur dünnflüssigen, klaren und geruchlosen Flüssigkeit, die einen Amin-Gehalt von 3.68 mmol NH₂/g aufwies. Das Produkt liegt grösstenteils in der offenkettigen (Aldimin-) Form vor.
IR: 2922, 2851, 1737 (C=O), 1668 (C=N), 1465, 1417, 1393, 1373, 1340, 1248, 1234, 1159, 1111, 1020, 1003, 933, 870, 722.
¹H-NMR (CDCl₃, 300 K): δ 7.52 (s, 2 H, CH=N), 4.02 (s, 4 H, CH₂O), 3.36 (*t*, 4 H, CH=NC*H*₂CH₂), 2.59 (*t*, 4 H, NHC*H*₂), 2.29 (*t*, 4 H, CH₂CO), 1.76-1.51 (m, 13 H, CH=NCH₂CH₂, NHCH₂CH₂, C*H*₂CH₂CO und CH₂N*H*CH₂), 1.27 (m, 40 H, CH₃-(C*H*₂)₈-CH₂CH₂CO und NHCH₂CH₂C*H*₂), 1.10 (s, 12 H, C(C*H*₃)₂-CH₂0), 0.88 (*t*, 6 H, C*H*₃-(CH₂)₁₀-CO).

### Aldimin AL3

In einem Rundkolben wurden unter Stickstoffatmosphäre 28.06 g (0.099 mol) 2,2-Dimethyl-3-lauroyloxy-propanal vorgelegt. Unter kräftigem Rühren wurden aus einem Eintropftrichter innerhalb von 3 Minuten 10.00 g (0.095 mol) 2-(2-Aminoethoxy)-ethanol (Diglycolamine^{®} Agent; Huntsman) zugegeben, wobei die Temperatur des Reaktionsgemisches auf 40°C anstieg. Danach wurden die flüchtigen Bestandteile im Vakuum entfernt (10 mbar, 80°C). Man erhielt 36.3 g einer farblosen, bei Raumtemperatur dünnflüssigen, klaren und geruchlosen Flüssigkeit, die einen Amin-Gehalt von 2.58 mmol NH₂/g aufwies. Das Produkt liegt grösstenteils in der offenkettigen (Aldimin-) Form vor.
IR: 3435br (O-H), 2954sh, 2922, 2852, 1736 (C=O), 1668 (C=N), 1466, 1418, 1394, 1375, 1248, 1233, 1160, 1127, 1062, 1022, 933, 893, 813, 721.
¹H-NMR (CDCl₃, 300 K): δ 7.59 (s, 1 H, CH=N), 4.03 (s, 2 H, CH₂O), 3.71 (m, 4 H, HOCH₂C*H*₂OC*H*₂CH₂N), 3.58 (m, 4 H, HOC*H*₂CH₂0CH₂C*H*₂N), 2.44 (br s, 1 H, *H*OCH₂), 2.30 (*t*, 2 H, CH₂CO), 1.61 (m, 2 H, C*H*₂CH₂CO), 1.26 (m, 16 H, CH₃-(C*H*₂)₈-CH₂CH₂CO), 1.11 (s, 6 H, C(C*H*₃)₂-CH₂O), 0.88 (*t*, 3 H, C*H*₃-(CH₂)₁₀-CO).

Das für die Herstellung der Aldimine ***AL1,** AL2* und ***AL3*** verwendete 2,2-Dimethyl-3-lauroyloxy-propanal wurde gemäss Beispiel 1 von WO 2004/013088 A1 hergestellt.

### Herstellung einer beispielhaften reaktiven Polyurethanzusammensetzung

Ein beispielhafter reaktiver Polyurethan-Heissschmelzklebstoff **K1** wurde durch wie folgt hergestellt:
800 g Dynacoll 7150 (Evonik, amorpher, gesättigter bei Raumtemperatur fester Polyester, Tg (DSC)= 50°C, 38-46 mg KOH/g) wurden mit 800 g Dynacoll 7250 (Evonik, bei Raumtemperatur flüssiger, gesättigter Polyester, Tg (DSC)= -50°C, OH-Zahl= 18-24 mg KOH/g) unter Stickstoff und Rühren in einem Rührkessel mit einem Ankerrührer unter Vakuum aufgeschmolzen. Nach 2 Stunden Rühren bei 130°C wurde unter Rühren hierauf 233 g Desmodur 44 MC Flakes(Bayer, 4,4'-Diphenylmethandiisocyanat (4,4'-MDI)) zugegeben. Nach 1 Stunden Rühren wurde Heissschmelzklebstoff abgefüllt. Der Klebstoff weist einen NCO-Gehalt von 2.2 % auf.

### Applikation

Für jeden der Versuche wurde nun wie folgt vorgegangen:
Der oben beschriebene reaktive Heissschmelzklebstoff **K1** wurde über eine Walzenanklage ICO WLA 1600 (ICO System, Deutschland) appliziert. Es wurde hierbei eine graphitierte Walze, ein Spalt von weniger als 1 mm und Klebstofftemperatur von 145°C verwendet. Die Auftragsgeschwindigkeit war 6 m/min. Die Drehgeschwindigkeit der Rakelwalze betrug 0.6 m/min.

Nachdem nun die Walzenanlage während 30 Minuten eingefahren war, wurde der aus der Walze austretende Klebstoff dazu verwendet um je 3 Zugscherproben herzustellen **(*Ref.1*)** bzw. *(*"*vor Reinigung")*

Die Zugscherprobenkörper wurden mit Hilfe einer Form hergestellt: Der Klebstoff wurde auf ein Plättchen getrockneten Buchenholz (100 mm x 25 mm x 5mm) aufgetragen, mit einem zweiten derartigen Buchenholplättchen auf eine Schichtdicke von 1 mm und 20 mm Überlappung mittels entsprechender Form in die Zugscherproben-Geometrie verpresst und während 7 Tagen bei 23 °C und 50 % relativer Luftfeuchtigkeit ausgehärtet.

Nun wurde die Zufuhr des Klebstoffs **K1** kurzfristig abgetrennt, jeweils pro kg des Klebstoffs 200 g des jeweiligen Aldimins bzw. der Alkohol gemäss Tabelle 1 als Reinigungsmittel in den Walzenspalt zugeführt. Es wurde kontinuierlich Proben genommen, um über IR das Verschwinden der Isocyanat-Gruppen zu untersuchen. Sobald keine Isocyanatgruppen mehr nachgewiesen werden konnten, hatte man die Gewissheit, dass nur noch blockierter Klebstoff auf der Walze vorhanden war. Nun wurde die Zufuhr des Klebstoffs **K1** wieder fortgesetzt, bis die Intensität der NCO-Bande in der IR-Messung 50% der ursprünglichen Intensität aufwies, so dass man die Gewissheit hatte, dass jetzt eine Mischung von blockiertem und nichtblockiertem Klebstoff **K1** gefördert wurde. Mit dem nun aus der Walze austretenden Klebstoff wurde nun je 3 weitere Zugscherprobenkörper *(*"*Mischfraktion")* hergestellt. Anschliessend wurde mit der Förderung des Klebstoff **K1** weitergefahren, bis die Intensität der Bande wieder den ursprünglichen Wert angenommen hatte, so dass man die Gewissheit hatte dass sich nun lediglich nicht-blockierter Klebstoff **K1** auf der Anlage befand. Mit dem nun aus der Walze austretenden Klebstoff wurde nun eine je 3 weitere Zugscherprobenkörper *(*"*nach Reinigung")* hergestellt.

Die Zugscherproben wurden nach der Aushärtung (7d, 23 °C und 50 % relativer Luftfeuchtigkeit) in vertikale Lage gebracht, fixiert und an das untere Holzplättchen ein Gewicht angehängt (statische Belastung 10 N) und in einen auf 170°C temperierten Umluftofen gestellt. Nach 1 Stunde wurde kontrolliert, ob die Zugscherprobenkörper intakt waren ("OK") oder ob der Probekörper zerstört wurde ("n.OK") und in Tabelle 1 eingetragen.

**Tabelle 1 Reinigungsmittel und Resultate.**

| | ***Ref.1*** | ***Ref.2*** | 1 | 2 | 3 |
|---|---|---|---|---|---|
| Reinigungsmittel | | | | | |
| ***AL1*** [g / kg Klebstoff] | | | 200 | | |
| ***AL2*** [g / kg Klebstoff] | | | | 200 | |
| ***AL3*** [g / kg Klebstoff] | | | | | 200 |
| Stearylalkohol [g / kg Klebstoff] | | 200 | | | |
| Belastungstest (*vor Reinigung*) | OK | OK | OK | OK | OK |
| Belastungstest (*Mischfraktion*) | | **n.OK** | OK | OK | OK |
| Belastungstest (*nach Reinigung*) | | OK | OK | OK | OK |

Es zeigt sich aus Tabelle 1, dass die Klebstoffe vor und nach der Reinigung **(*Ref.1*)** eine tadellose Haftung und gute Mechanik aufweisen. Bei den durch das Reinigungsmittel blockierten Klebstoffen vermengten Mischfraktionen hingegen zeigt sich, dass bei der Blockierung durch Stearylalkohol (ein Blockierungsmittel, wie es in Reinigungsmitteln des Stand der Technik verwendet wird) **(*Ref.2*),** Verklebungen resultieren, welche Schwächen in der Wärmebeständigkeit aufweisen, während dies bei den Beispielen **1, 2** und **3** nicht der Fall ist.

Somit können in den Beispielen **1, 2** und **3** auch Mischfraktionen von blockierten und unblockierten Klebstoffen verwendet werden, um verlässliche Verklebungen herzustellen, während dies beim Vergleichsreinigungsmittel ***Ref. 2*** nicht der Fall ist.

### Verwendung von Reinigungsmitteln zur Reinigung

In einem weiteren Versuch wurden bei der oben beschriebenen Walzenanlage nach Dauerbetrieb mit dem reaktiven Heissschmelzklebstoffs **K1** Verkrustungen an der Walze insbesondere bzw. an den Rändern und der Rakelwalze festgestellt. Nach der Beschickung der Anlage -wie oben beschrieben- mit Reinigungsmittel ***AL1*** wurden die Verunreinigungen selbstständig entfernt. Zuweilen war ein Nachwischen mittels eines Tuches nötig.

Nachdem -wie oben über IR-Messung beschrieben wurde- die Abwesenheit von NCO-Gruppen in dem aus der Walze austretende Klebstoff bestätigt wurde, konnte eine so gereinigte Walzenanlage problemlos übers Wochenende abgeschaltet werden und danach ohne Probleme am Montag wieder in Betrieb genommen werden. Es zeigten sich nirgends auch nur Spuren von Anhärtungen oder Verkrustungen.

Entsprechendes verhalten zeigte sich auch bei den Reinigungsmitteln ***AL2*** und ***AL3***. Wurde kein Reinigungsmittel verwende, waren die Verkrustungen und Verklebungen der Walze derart gross, dass nach dem Wochenende die Anlage nicht in Betrieb genommen werden konnte, sondern eine arbeitintensive Überholung und starke und aufwändige Reinigung nötig wurde.

## Patentansprüche

1. Verwendung eines Aldimins der Formel (I) als Reinigungsmittel oder als Bestandteil einer Reinigungsmittelzusammensetzung für reaktive Polyurethanzusammensetzungen, insbesondere für reaktive Polyurethan-heissschmelzklebstoffe, 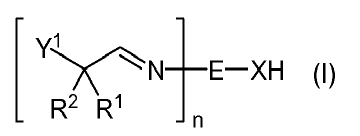 wobei
R¹ und R² entweder
unabhängig von einander jeweils für einen einwertigen Kohlenwasserstoffrest mit 1 bis 12 C-Atomen stehen,
oder
zusammen für einen zweiwertigen Kohlenwasserstoffrest mit 4 bis 12 C-Atomen, der Teil eines, gegebenenfalls substituierten, carbocyclischen Rings mit 5 bis 8, bevorzugt 6, C-Atomen ist, stehen;
Y¹ für einen einwertigen Kohlenwasserstoffrest mit 1 bis 32 C-Atomen steht, welcher gegebenenfalls mindestens ein Heteroatom, insbesondere Sauerstoff in der Form von Ether-, Carbonyl- oder Ester-Gruppen aufweist; und
E für einen (n+1)-wertigen Kohlenwasserstoffrest mit 2 bis 12 C-Atomen, welcher gegebenenfalls mindestens ein Heteroatom, insbesondere in Form von Ether-Sauerstoff oder tertiärem Amin-Stickstoff, aufweist, steht; X für O, S oder N-R⁸ steht
wobei
R⁸ entweder
für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureestergruppe aufweist, steht,
oder
für einen Substituenten der Formel (II) steht 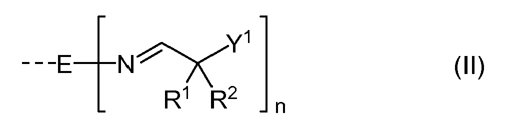 und n für 1, 2 oder 3 steht.

2. Verwendung gemäss Anspruch 1, **dadurch gekennzeichnet, dass** R¹ und R² je für einen Methylrest stehen.

3. Verwendung gemäss Anspruch 1 oder 2, **dadurch gekennzeichnet, dass** Y¹ für einen Rest der Formel (III a) oder (III b) steht. 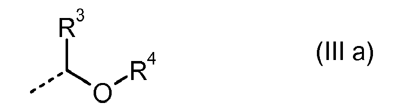 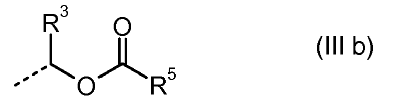 wobei
R³ für ein Wasserstoffatom oder für einen Alkyl-, Cycloalkyl-, Arylalkyl-oder Alkoxycarbonylrest mit 1 bis 12 C-Atomen steht;
R⁴ für einen Kohlenwasserstoffrest mit 1 bis 30 C-Atomen, welcher gegebenenfalls Ethersauerstoffatome enthält, steht;
R⁵ entweder
für ein Wasserstoffatom steht,
oder
für einen linearen oder verzweigten Alkylrest mit 1 bis 30 C-Atomen, gegebenenfalls mit cyclischen Anteilen und gegebenenfalls mit mindestens einem Heteroatom, insbesondere Sauerstoff in der Form von Ether-, Carbonyl- oder Ester-Gruppen, steht,
oder
für einen einwertigen einfach oder mehrfach ungesättigten, linearen oder verzweigten Kohlenwasserstoffrest mit 5 bis 30 C-Atomen steht,
oder
für einen, gegebenenfalls substituierten, aromatischen oder heteroaromatischen 5- oder 6-gliedrigen Ring steht.

4. Verwendung gemäss Anspruch 3, **dadurch gekennzeichnet, dass** Y¹ für den Rest der Formel (III a) steht und R⁴ für einen linearen oder verzweigten Alkylrest mit 6 bis 30, insbesondere mit 11 bis 30, C-Atomen, steht.

5. Verwendung gemäss Anspruch 3, **dadurch gekennzeichnet, dass** Y¹ für den Rest der Formel (III b) steht und R⁵ für einen linearen oder verzweigten Alkylrest mit 6 bis 30, insbesondere mit 11 bis 30, C-Atomen, steht.

6. Verwendung gemäss einem der Ansprüche 3 bis 5, **dadurch gekennzeichnet, dass** R³ für H steht.

7. Verwendung gemäss einem der vorhergehenden Ansprüche, **dadurch gekennzeichnet, dass** der Substituent E 3 bis 6 C-Arome aufweist.

8. Verwendung gemäss einem der vorhergehenden Ansprüche, **dadurch gekennzeichnet, dass** das Aldimin der Formel (I) die Struktur der Formel (III b-1) oder (III b-2) oder (III b-3) oder (III b-4) aufweist 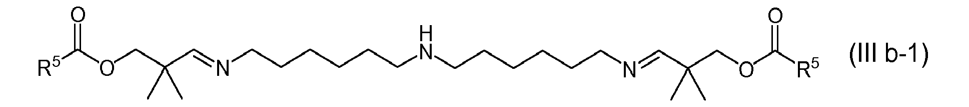 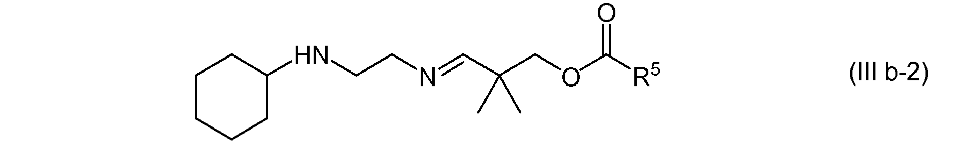 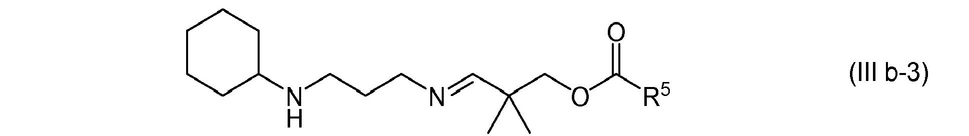 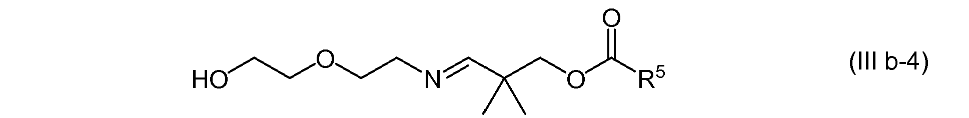 Wobei R⁵ entweder
für ein Wasserstoffatom steht,
oder
für einen linearen oder verzweigten Alkylrest mit 1 bis 30 C-Atomen, gegebenenfalls mit cyclischen Anteilen und gegebenenfalls mit mindestens einem Heteroatom, insbesondere Sauerstoff in der Form von Ether-, Carbonyl- oder Ester-Gruppen, steht,
oder
für einen einwertigen einfach oder mehrfach ungesättigten, linearen oder verzweigten Kohlenwasserstoffrest mit 5 bis 30 C-Atomen steht,
oder
für einen, gegebenenfalls substituierten, aromatischen oder heteroaromatischen 5- oder 6-gliedrigen Ring steht.

9. Verwendung gemäss einem der vorhergehenden Ansprüche, **dadurch gekennzeichnet, dass** n für den Wert 1 steht.

10. Reinigungsmittelzusammensetzung für reaktive Polyurethanzusammensetzungen, insbesondere für reaktive Polyurethanheissschmelzklebstoffe umfassend
- mindestens ein Aldimin der Formel (I), wie es in der Verwendung gemäss einem der Ansprüche 1 bis 9 beschrieben ist;
sowie
- mindestens ein Trägermittel **A**, welches keine gegenüber Isocyanat reaktive funktionellen Gruppen trägt.

11. Reinigungsmittelzusammensetzung gemäss Anspruch 10, **dadurch gekennzeichnet, dass** das Trägermittel **A** ein bei Raumtemperatur festes thermoplastisches Trägermittel **A** ist, vorzugsweise ein blockiertes Polyurethanprepolymer, ein Kohlenwasserstoffharz, ein blockierter Polyester, ein blockiertes Polyol oder ein Copolymer aus mindestens einem der Monomere, welche ausgewählt sind aus der Gruppe bestehend aus Ethylen, Propylen, Butylen, Butadien, Vinylester, Vinylchlorid, Styrol, Ester der Acrylsäure und Ester der Methacrylsäure, ist.

12. Reinigungsmittelzusammensetzung gemäss Anspruch 10 oder 11, **dadurch gekennzeichnet, dass** das Trägermittel **A** ein Ethylen/Vinylacetat-Copolymer (EVA) ist.

13. Reinigungsmittelzusammensetzung gemäss einem der Ansprüche 10 bis 13, **dadurch gekennzeichnet, dass** die Reinigungsmittelzusammensetzung weiterhin mindestens einen Katalysator für die Reaktion der Gruppe HX mit Isocyanat, insbesondere eine Organozinnverbindung und/oder eine Bismutverbindungen und/oder ein tertiäres Amin, enthält.

14. Verfahren zur Reinigung einer Herstell- oder Applikationsanlage oder eines Applikationsgerätes von reaktiven Polyurethanzusammensetzungen, **dadurch gekennzeichnet, dass**
ein Aldimin der Formel (I), wie es in der Verwendung gemäss einem der Ansprüche 1 bis 9 beschrieben ist,
oder eine Reinigungsmittelzusammensetzung gemäss einem der Ansprüche 10 bis 13
zu einer in dieser Anlage oder Gerät befindlichen reaktiven Polyurethanzusammensetzung zugegeben wird und diese aus der Anlage oder dem Gerät verdrängt wird.

15. Verfahren gemäss Anspruch 14, **dadurch gekennzeichnet, dass** die reaktive Polyurethanzusammensetzung ein reaktiver Polyurethan-Heissschmelzklebstoff ist.
